# FASCICULE DE BREVET EUROPEEN

(11) **EP 2 699 187 B1**
(45) Date de publication et mention de la délivrance du brevet: **02.10.2019**
(21) Numéro de dépôt: 12722437.6
(22) Date de dépôt: 19.04.2012
(51) Int. Cl.: A61B 50/30, A61B 17/86, A61F 2/00

(54) **ENSEMBLE MEDICAL COMPRENANT UN OBJET MEDICAL ET UN EMBALLAGE CONTENANT LEDIT OBJET**
MEDIZINISCHE ANORDNUNG MIT EINEM MEDIZINISCHEN ARTIKEL UND PAKET MIT DIESEM ARTIKEL
MEDICAL ASSEMBLY COMPRISING A MEDICAL ARTICLE AND A PACKAGE CONTAINING SAID ARTICLE

(30) Priorité: 22.04.2011 FR 1153520
(43) Date de publication de la demande: 26.02.2014
(73) Titulaire: Synthes GmbH, 4436 Oberdorf (CH)
(72) Inventeur: RICHART, Olivier, F-17140 Lagord (FR); LARCHE, Grégoire, F-49300 Cholet (FR); PODGORSKI, Jean-Pierre, F-49230 Saint Crespin Sur Moine (FR)
(74) Mandataire: Klunker IP Patentanwälte PartG mbB
(86) Numéro de dépôt international: PCT/FR2012/050852
(87) Numéro de publication internationale: WO 2012/172215

(56) Documents cités:
- EP-A1- 1 523 955
- FR-A1- 2 581 867
- US-A- 4 856 648
- US-A- 5 062 800
- US-A1- 2009 065 387

## Description

La présente invention concerne de manière générale les emballages pour objets, en particulier pour pièces médicales de préférence stérilisées.

L'invention concerne plus particulièrement un ensemble médical, de préférence stérilisé, comprenant un objet médical et un emballage contenant ledit objet.

On connaît de l'état de la technique des emballages se présentant sous la forme de double enveloppes externe et interne de type sachets ou double coques plastiques blister ou d'une combinaison des deux dans lesquels un objet est contenu. Dans le cas de pièces médicales devant être déconditionnées dans des conditions proches de l'asepsie, de tels emballages peuvent poser des problèmes de contamination lors du passage de l'emballage d'une personne à une autre et de l'ouverture dudit emballage. En effet, depuis son départ d'une zone de travail dite sale, c'est-à-dire sans condition d'asepsie particulière, jusqu'à son arrivée dans une zone de travail dite propre, c'est-à-dire une zone de travail dans laquelle des conditions d'asepsie données sont respectées, par exemple la zone « stérile » du bloc opératoire, l'emballage qui arrive en zone propre est contaminé au niveau de son enveloppe extérieure, ce qui présente un risque de contamination de la pièce médicale en sortant celle-ci de l'emballage.

Pour limiter le risque de contamination de l'objet contenu dans l'emballage, il est connu d'ouvrir ledit emballage de manière à faire tomber l'objet sur un plan de travail pour ne pas avoir à toucher ledit objet. Seul le chirurgien ou l'assistant stérile prend l'objet. Cependant, l'objet risque non seulement de se détériorer sous le choc avec le plan de travail, dans le cas d'un objet solide, ou encore de tomber par terre en roulant, mais également d'être contaminé par des corps étrangers présents sur le plan de travail. Il arrive également que l'opérateur tende l'enveloppe extérieure de l'emballage à l'assistant stérile qui doit alors essayer de prendre au mieux l'enveloppe interne sans commettre de faute d'asepsie. Cependant, le risque que l'assistant stérile touche une zone contaminée de l'emballage au cours de cette opération et que l'objet saisi soit à son tour contaminé est très important.

Le document US5062800 décrit un emballage médical pour implant dentaire qui comprend un élément support muni de moyens de fixation de l'implant et un bouchon couplable à l'élément support de telle sorte que l'élément support s'étend en saillie dudit bouchon. Ledit emballage comprend également une coiffe qui recouvre le bouchon. Cependant, la coiffe recouvre entièrement la longueur du bouchon de sorte que l'opérateur n'a pas d'autre solution que de vider le contenu de la coiffe en le faisant tomber sur un plan de travail. Comme rappelé ci-dessus, le fait de faire tomber l'implant, éventuellement avec son enveloppe, peut détériorer ledit implant et augmente le risque de contamination.

En outre, dans le document US5062800, l'implant est fixé à l'élément support correspondant de l'emballage. Il en résulte que l'opérateur doit réaliser une manipulation spécifique pour extraire l'implant de l'élément support une fois les autres éléments retirés, ce qui complique l'intervention de l'opérateur et ne lui permet pas de saisir rapidement l'implant dont il a besoin. En particulier, dans le domaine urgentiste ou de la traumatologie, l'opérateur qui a besoin dudit implant doit pouvoir en disposer le plus rapidement possible, tout en limitant bien entendu le risque de contamination dudit implant.

Le document FR2581867 décrit un dispositif d'emballage de produits pour la prise de doses homéopathiques, qui comprend un récipient tubulaire transparent renfermant une dose de produit et qui est disposé dans un étui protecteur opaque fermé par un bouchon opaque constituant également un organe obturateur du récipient tubulaire.

Le document EP1523955 concerne un emballage de conservation d'un dispositif médical comprenant une capsule et un capuchon coopérant de façon étanche avec la capsule.

La présente invention a pour but de proposer un emballage pour le conditionnement et le déconditionnement d'objet qui permet de limiter les risques de contamination de l'objet tout en permettant un accès aisé et rapide audit objet.

L'ensemble médical, de préférence stérilisé, comprenant un objet médical et un emballage selon la présente invention est définit par la revendication 1.

La présente invention concerne également un procédé de déballage d'un objet médical selon la revendication 10.

Les modes de réalisation préférés sont définis dans les revendications dépendantes.

A cet effet, l'invention a pour objet un ensemble médical, de préférence stérilisé, comprenant un objet médical et un emballage contenant ledit objet, ledit emballage comprenant un élément support creux à l'intérieur duquel ledit objet est libre,
caractérisé en ce que ledit emballage comprend :
- un premier élément de protection, appelé bouchon, couplable à l'élément support dudit objet, de préférence par emboitement à recouvrement partiel, de telle sorte que l'élément support présente une partie de saisie qui s'étend en saillie dudit bouchon,
   ledit élément support délimitant à lui seul ou en coopération avec le bouchon une première chambre renfermant ledit objet,
- un deuxième élément de protection creux, appelé coiffe,
ladite coiffe étant couplable au bouchon, de préférence par emboitement à recouvrement partiel, de manière à délimiter en coopération avec ledit bouchon une deuxième chambre à l'intérieur de laquelle s'étend la partie de saisie de l'élément support,
et en ce que, à l'état couplé de la coiffe au bouchon, une partie du bouchon s'étend en saillie de la coiffe.

Grâce à la partie de saisie de chaque élément de protection et de l'élément support, qui s'étend en saillie de l'élément de protection avec lequel il est couplé, les chambres définies par emboitement à recouvrement partiel desdits éléments peuvent être ouvertes jusqu'à accéder à l'objet sans jamais lâcher ou toucher ledit objet et avec un risque réduit de contamination de l'objet.

En effet lors du passage de l'emballage d'une première personne dont les mains sont supposées "contaminées" à une deuxième personne dont les mains sont supposées "propres", la coiffe de protection peut être retirée par la première personne de manière à ouvrir la "deuxième" chambre pour découvrir l'extrémité de saisie de l'élément support de l'objet et ne conserver pour la deuxième personne que le sous-ensemble formé du bouchon et de l'élément support.

La première personne tient ledit sous-ensemble par l'extrémité du bouchon opposée à l'élément support et peut tendre l'extrémité propre de l'élément support, découverte par l'ouverture de la première chambre, à la deuxième personne, par exemple le chirurgien.

Le bouchon et l'élément support peuvent alors être séparés de manière à ouvrir la chambre contenant l'objet. La deuxième personne, qui ne conserve ainsi que l'élément support qui contient l'objet, peut alors de sa main propre qui tient l'extrémité de saisie de l'élément support, saisir l'objet par exemple par son autre main propre.

Une telle conception de l'emballage selon l'invention permet de réaliser un emballage avec un nombre de pièces réduit, principalement trois, de telle sorte que, lors du passage de l'emballage depuis une zone sale jusqu'à une zone propre par l'intermédiaire de différentes personnes, ni l'élément support ni l'objet ne sont touchés par une personne en zone sale et l'objet contenu dans l'élément support n'est jamais lâché. Le risque de contamination est ainsi très fortement réduit.

En outre, le fait que l'objet soit libre à l'intérieur de l'élément support permet un accès aisé et rapide audit objet. En particulier, dans le domaine urgentiste, la personne qui a besoin dudit objet peut disposer rapidement dudit objet, tout en limitant le risque de contamination dudit objet comme détaillé ci-après.

Selon l'invention, ledit bouchon comprend une paroi périphérique dite interne, et une paroi périphérique dite externe qui entoure à écartement ladite paroi périphérique interne pour définir entre lesdites parois périphériques un espace annulaire d'emboitement de la partie de la coiffe destinée à être couplée audit bouchon, la paroi périphérique interne définissant un espace d'emboitement de la partie de l'élément support destinée à être couplée audit bouchon.

Selon l'invention, la face interne de la paroi périphérique interne du bouchon est munie de moyens de maintien permettant de maintenir la partie de l'élément support emboitée à l'intérieur de l'espace délimité par ladite paroi périphérique interne du bouchon.

Selon l'invention, lesdits moyens de maintien comprennent des ergots qui sont destinés à être en contact d'appui avec l'élément support à l'état emboité dudit élément support dans le bouchon, et les ergots sont répartis sur la face périphérique interne du bouchon, autour de l'axe dudit bouchon, en étant espacés angulairement les uns des autres.

Selon une caractéristique avantageuse de l'invention, chaque ergot comprend une partie formant rampe, dirigée vers l'axe du bouchon en partant d'un point bas de la rampe situé du côté de l'extrémité ouverte du bouchon vers un point haut de ladite rampe situé du côté de l'extrémité fermée dudit bouchon, de sorte que, lors de l'emboitement de l'élément support à l'intérieur de l'espace défini par la paroi périphérique interne du bouchon, ledit élément support, par appui sur ladit rampe, pousse radialement ledit ergot et déforme la paroi périphérique interne correspondante vers la paroi périphérique externe.

Selon une caractéristique avantageuse de l'invention, les ergots forment des entretoises permettant de maintenir un écartement radial entre l'élément support et la paroi périphérique interne du bouchon, l'espacement entre les ergots définissant un espace de communication de fluide entre l'intérieur de la paroi périphérique du bouchon et l'intérieur de la coiffe.

Selon une caractéristique avantageuse de l'invention, lesdites parois périphériques externe et/ou interne du bouchon comprennent des moyens de vissage aptes à coopérer avec des moyens de vissage complémentaires ménagés sur la coiffe.

Selon une caractéristique avantageuse de l'invention, la face interne de la paroi périphérique du bouchon externe présente un taraudage apte à coopérer avec un filet ménagé sur la face externe de la paroi périphérique de la coiffe.

Selon une caractéristique avantageuse de l'invention, ledit élément support délimitant à lui seul ladite première chambre renfermant ledit objet, ledit élément support est formé d'au moins deux pièces couplables/désaccouplables l'une par rapport à l'autre, de préférence par emboitement à recouvrement partiel, pour former ladite première chambre renfermant ledit objet.

Ainsi, la conception de l'emballage sous la forme d'un élément support couplé à un bouchon qui délimite en coopération avec une coiffe une chambre dans laquelle une partie de l'élément support s'étend en saillie du bouchon, permet de faire passer l'élément support d'une première personne à une deuxième personne sans que la première personne ne touche l'élément support et avec un risque réduit de contamination entre la première et la deuxième personne, de sorte que l'élément support qui renferme ledit objet arrive en zone propre avec un risque réduit de contamination. En outre, la conception de l'élément support sous la forme de deux pièces, couplables pour délimiter une cavité fermée (première chambre), permet de protéger ledit objet en attente d'utilisation.

Selon une caractéristique avantageuse de l'invention, ledit élément support délimitant en coopération avec le bouchon ladite première chambre renfermant ledit objet, ledit élément support délimite une cavité ouverte destinée à être refermée par ledit bouchon.

Selon un tel mode de réalisation, ledit emballage peut être formé de seulement trois pièces, à savoir la coiffe, le bouchon, et l'élément support. Chaque pièce se présente sous la forme d'un corps creux ouvert à la manière d'une éprouvette. Ainsi, l'élément support est couplable au bouchon pour délimiter la première chambre qui renferme ledit objet et le bouchon est couplable à la coiffe pour délimiter la deuxième chambre qui renferme la partie de l'élément support qui s'étend en saillie du bouchon.

Selon une caractéristique avantageuse de l'invention, le bouchon et la coiffe sont chacun formés par un corps creux allongé ouvert à une extrémité et fermé à l'autre extrémité.

Le caractère allongé de l'un ou des éléments de protection de l'objet formés par la coiffe et le bouchon permet de bénéficier d'une distance de sécurité lors de l'ouverture des chambres au cours du passage des différentes parties de l'emballage d'une personne à une autre.

Selon une caractéristique avantageuse de l'invention, ledit bouchon, et éventuellement la coiffe, présente(nt) des emplacements prédéfinis permettant de positionner au moins deux doigts, de préférence trois doigts, d'une personne pour lui permettre de saisir ledit bouchon, et éventuellement ladite coiffe, au niveau desdits emplacements prédéfinis.

Avantageusement, ledit bouchon, et éventuellement ladite coiffe, présente(nt) des emplacements prédéfinis permettant de positionner au moins trois doigts pour saisir par pincement ledit bouchon, et éventuellement ladite coiffe.

Un tel emplacement prédéfini permet de limiter le risque de contamination de l'élément support et donc de l'objet contenu dans ledit élément support.

Selon une caractéristique avantageuse de l'invention, ladite coiffe étant couplable au bouchon par emboitement à recouvrement partiel, ledit bouchon présente au moins une partie, de préférence au moins une ailette, déformable configurée pour, d'une part, à l'état recouvert de ladite partie déformable du bouchon par la coiffe, venir en appui contre la face interne de coiffe afin de permettre un couplage du bouchon à la coiffe par friction et, d'autre part, pour pouvoir se déformer afin de permettre la séparation de la coiffe du bouchon.

Une telle partie déformable du bouchon assure une bonne étanchéité entre le bouchon et la coiffe à l'état couplé de ces deux pièces.

Selon une caractéristique avantageuse de l'invention, lesdits éléments de support et de protection de l'emballage s'emboitent à recouvrement partiel entre eux par friction et/ou par vissage.

Selon une caractéristique avantageuse de l'invention, l'élément support et/ou le bouchon et/ou la coiffe présente(nt) une partie, de préférence formée par une membrane, imperméable aux bactéries mais perméable aux gaz pour permettre une stérilisation gazeuse de ladite première chambre et/ou de ladite deuxième chambre.

L'invention concerne également un procédé de déballage d'un objet médical contenu dans un emballage d'ensemble médical, de préférence stérilisé, tel que décrit ci-dessus, caractérisé en ce que ledit procédé comprend les étapes suivantes :
a) - séparation de la coiffe par rapport au bouchon par une première personne, dite personne contaminée, de manière à découvrir l'élément support,
b) - saisie par une deuxième personne, dite personne non contaminée, de l'élément support,
c) - séparation dudit élément support par rapport au bouchon tenu par ladite personne contaminée.

Selon une caractéristique avantageuse de l'invention, ledit procédé comprend les étapes supplémentaires suivantes :
d) - éventuellement, mise en attente dudit élément support qui renferme l'objet dans une zone d'attente, en attente de l'utilisation dudit objet,
e) - ouverture de la première chambre définie par ledit élément support pour en extraire ledit objet.

Ces étapes de procédé s'appliquent en particulier à un mode de réalisation de l'emballage selon l'invention, pour lequel l'élément support forme à lui seul ladite première chambre et pour lequel ledit élément support est formé d'au moins deux pièces couplables/désaccouplables l'une à l'autre qui, à l'état couplé, délimitent une cavité fermée formant ladite première chambre. La première personne tend l'emballage restant formé du bouchon et de l'élément support à la deuxième personne en lui présentant l'extrémité dudit emballage restant formée par ledit élément support. La deuxième personne tire alors sur ledit élément support de manière à le séparer dudit bouchon. L'élément support qui forme une cavité qui renferme l'objet peut être posé sur une table dans une zone dite décontaminée en attendant d'être utilisé. La réalisation de l'élément support sous forme d'une cavité qui renferme l'objet permet de protéger ledit objet qui reste, à l'intérieur dudit élément support, en attente d'utilisation contre tout risque de contamination extérieure. Il suffit alors à la deuxième personne, ou à une autre personne "propre" d'ouvrir la première chambre formée par ledit élément support en séparant au moins les deux pièces qui forment ledit élément support.

Selon une caractéristique avantageuse de l'invention, ledit procédé comprend entre les étapes a) et b) l'étape supplémentaire suivante de présentation, par la première personne à la deuxième personne, de l'emballage restant, formé du bouchon et de l'élément support, en lui présentant l'extrémité dudit emballage restant formée par ledit élément support, en orientant ledit emballage restant de telle sorte que l'élément support se retrouve à une hauteur inférieure à celle du bouchon de sorte que ledit objet reste contenu dans l'élément support lors de la séparation dudit élément support par rapport au bouchon pour éviter que ledit objet ne tombe par terre.

Cette étape du procédé s'applique en particulier à un mode de réalisation de l'emballage selon l'invention pour lequel l'élément support forme ladite première chambre en coopération avec le bouchon et ledit élément support se présente sous la forme d'un corps creux allongé, ouvert à une extrémité et fermé à l'autre extrémité, apte à contenir ledit objet. La première personne dite "contaminée" tend l'emballage restant formé du bouchon et de l'élément support à la deuxième personne dite "propre" ou "stérile" en lui présentant l'extrémité dudit emballage restant formée par ledit élément support, en orientant ledit emballage restant de telle sorte que l'élément support se retrouve à une hauteur inférieure à celle du bouchon de sorte que ledit objet reste contenu dans l'élément support lors de la séparation dudit élément support par rapport au bouchon pour éviter que ledit objet ne tombe par terre. La deuxième personne, ou une autre personne propre, peut alors saisir, éventuellement à l'aide d'un outil, l'objet en contact avec ledit élément support.

L'invention sera bien comprise à la lecture de la description suivante d'exemples de réalisation, en référence aux dessins annexés dans lesquels :
- la figure 1 est une vue d'un emballage selon l'invention conformément à un premier mode de réalisation, à l'intérieur duquel est conditionnée une pièce médicale ;
- les figures 2 à 4 sont des vues de l'emballage de la figure 1 au cours d'une première étape de déconditionnement pour laquelle la coiffe est retirée par une première personne dite personne contaminée, le sous-ensemble restant formé du bouchon et du support de pièce étant maintenu côté bouchon par la main de la personne contaminée ;
- les figures 5 et 6 sont des vues du sous-ensemble de l'emballage de la figure 4 au cours d'une deuxième étape de déconditionnement pour laquelle le bouchon est retiré en restant dans la main de la personne contaminée, et l'élément support de l'objet est tenu par la main d'une deuxième personne dite personne propre ;
- la figure 7 est une vue de l'élément support de la figure 5 au cours d'une troisième étape de déconditionnement pour laquelle la personne propre sépare les deux pièces qui forment l'élément support pour en extraire la pièce médicale ;
- la figure 8 est une vue en perspective du bouchon de l'emballage conforme au premier mode de réalisation de l'invention;
- la figure 8A est une vue en perspective d'une variante de réalisation du bouchon de l'emballage selon l'invention avec une zone de positionnement de doigts dite externe ménagée sur la partie du bouchon destinée à s'étendre en saillie de la coiffe;
- la figure 8B est une vue en perspective d'une autre variante de réalisation du bouchon de l'emballage selon l'invention avec une zone de positionnement de doigts dite interne ménagée sur la partie du bouchon destinée à s'étendre à l'intérieur de la coiffe.

On entend par personne "sale" ou "contaminée" une personne travaillant dans des conditions non aseptisées, qui est susceptible de contaminer par ses mains les objets qu'elle touche. A l'inverse, on entend par personne "propre" une personne travaillant dans des conditions d'asepsie suffisante.

En référence aux figures et comme rappelé ci-dessus, l'invention concerne un ensemble médical comprenant un objet 5 médical et un emballage 1 contenant ledit objet 5 à des fins de préservation du caractère stérile de l'objet et en vue du déballage dudit objet dans des conditions aseptiques ou proches de l'asepsie. Ledit objet peut être par exemple une pièce solide, telle qu'une vis pour chirurgie, ou tout autre type d'objet, en particulier tout autre type d'implant. En outre, ledit objet peut être un liquide ou une poudre. Ledit objet et, de préférence, les différentes parties de l'emballage, sont stérilisés comme détaillé ci-après.

Ledit emballage comprend un élément support 2 creux à l'intérieur duquel ledit objet 5 est libre. Autrement dit, ledit objet 5 peut se déplacer librement à l'intérieur de l'élément support 2. Ledit emballage comprend également un premier élément de protection 3, appelé bouchon, couplable à l'élément support 2 dudit objet 5 par emboitement à recouvrement partiel, de telle sorte que l'élément support 2 présente une partie 20 de saisie qui s'étend en saillie dudit bouchon 3. Ladite partie 20 de saisie correspond à la partie d'extrémité libre de l'élément support 2 qui s'étend en saillie dudit bouchon 3. Comme détaillé ci-après, ledit élément support 2 délimite à lui seul ou en coopération avec le bouchon 3 une première chambre 7 renfermant ledit objet 5.

Ledit emballage comprend également un deuxième élément de protection 4 creux, appelé coiffe. Ladite coiffe 4 est couplable au bouchon 3, par emboitement à recouvrement partiel, de manière à délimiter en coopération avec ledit bouchon 3 une deuxième chambre 8 à l'intérieur de laquelle s'étend l'extrémité de saisie 20 de l'élément support 2. A l'état couplé de la coiffe 4 au bouchon 3, une partie 30 du bouchon 3 s'étend en saillie de la coiffe 4.

Ladite partie 30 du bouchon correspond à l'extrémité libre du bouchon 3, opposée à l'extrémité 31 dudit bouchon 3 qui recouvre une partie de l'élément support 2 et forme une zone de saisie. L'extrémité 40 de la coiffe 4 opposée au bouchon 3 forme également une partie de saisie de l'emballage opposée à l'extrémité 30 du bouchon.

Ainsi, chaque élément de l'emballage, à savoir l'élément support 2, le bouchon 3 et la coiffe 4, présente une extrémité de saisie 20, 30, 40 par rapport à son extrémité opposée couplée avec un autre élément de l'emballage. Lesdits éléments s'emboitent entre eux de manière à définir, par coopération de deux éléments emboités, une chambre 7, 8 de préférence étanche au moins aux bactéries. Ainsi, les éléments emboités de l'emballage définissent deux chambres 7, 8 dont une 7, définie entre le bouchon et la coiffe, à l'intérieur de laquelle est logé l'objet et à laquelle on ne peut accéder qu'une fois ouverte l'autre chambre 8 servant de protection de l'extrémité de saisie 20 de l'élément support 2.

Une telle conception de l'emballage permet de le manipuler sans risque de contamination de l'objet contenu dans la chambre de présentation, sans avoir à lâcher ledit objet contenu dans l'élément support, tout en permettant de saisir rapidement ledit objet puisque celui-ci est libre dans l'élément support.

Dans l'exemple illustré aux figures, ledit élément support 2 délimite à lui seul ladite première chambre 7 renfermant ledit objet 5. A cet effet, ledit élément support 2 est formé d'au moins deux pièces 20, 21 couplables/ désaccouplables l'une par rapport à l'autre, par emboitement à recouvrement partiel, pour former ladite première chambre renfermant ledit objet 5.

On peut également prévoir un mode de réalisation selon lequel ledit élément support 2 délimite à lui seul ladite première chambre 7 en étant formé de manière monobloc. Ledit élément support 2 peut ainsi se présenter sous la forme d'un corps creux fermé monobloc sécable, tel qu'une ampoule sécable, contenant ledit objet. Dans ce cas, l'ouverture de la chambre 7 s'effectue en cassant ladite ampoule.

Selon une variante de réalisation non illustrée, ledit élément support délimite en coopération avec le bouchon ladite première chambre renfermant ledit objet. Dans ce cas, ledit élément support délimite une cavité ouverte destinée à être refermée par ledit bouchon.

Préférentiellement, le bouchon 3 est formé par un corps creux allongé ouvert à une extrémité et fermé à l'autre extrémité. Dans l'exemple illustré aux figures, la coiffe 4 est également formée par un corps creux allongé ouvert à une extrémité et fermé à l'autre extrémité. L'élément support 2 se présente également sous la forme d'un corps creux allongé fermé à une extrémité et ouverte à l'autre extrémité, ou fermé à ses deux extrémités et formé d'au moins deux pièces, suivant le mode de réalisation choisi.

La conception du bouchon 3 et de la coiffe 4 sous forme d'un corps allongé à la manière d'une éprouvette ou d'un tube fermé à au moins une extrémité, permet de bénéficier, d'une part, d'une distance de sécurité entre l'extrémité de saisie 40 de la coiffe 4 et l'extrémité de saisie 30 du bouchon 3 et, d'autre part, d'une distance de sécurité entre l'extrémité de saisie 20 de l'élément support 2 et l'extrémité de saisie 30 du bouchon 3.

Une telle distance entre les extrémités de saisie 30, 40 de l'emballage permet de limiter le risque de contamination au niveau de l'extrémité de saisie 20 de l'élément support 2 qui s'étend dans la chambre 8, lorsque ladite chambre 8 est ouverte par séparation de la coiffe 4 et du bouchon 3.

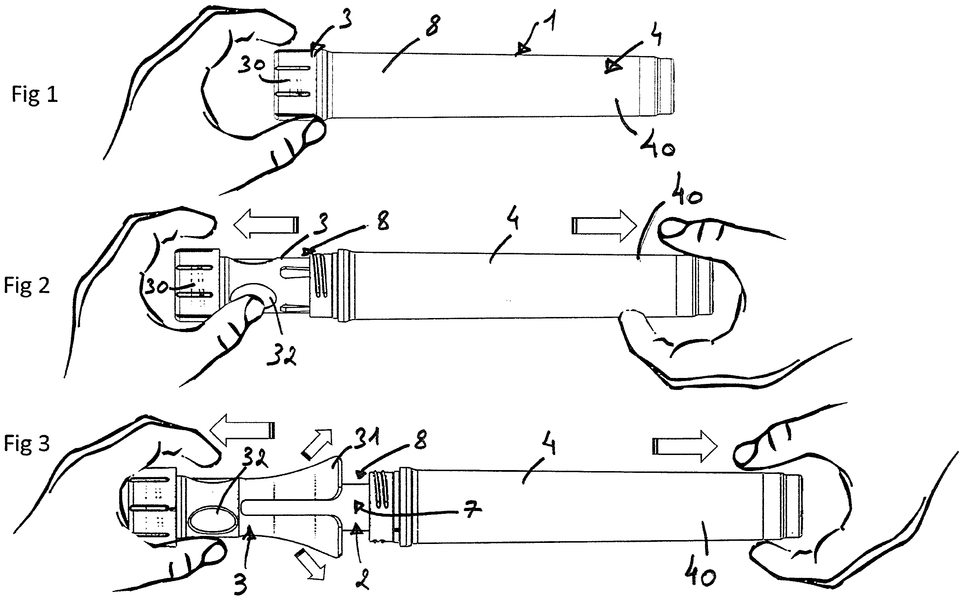

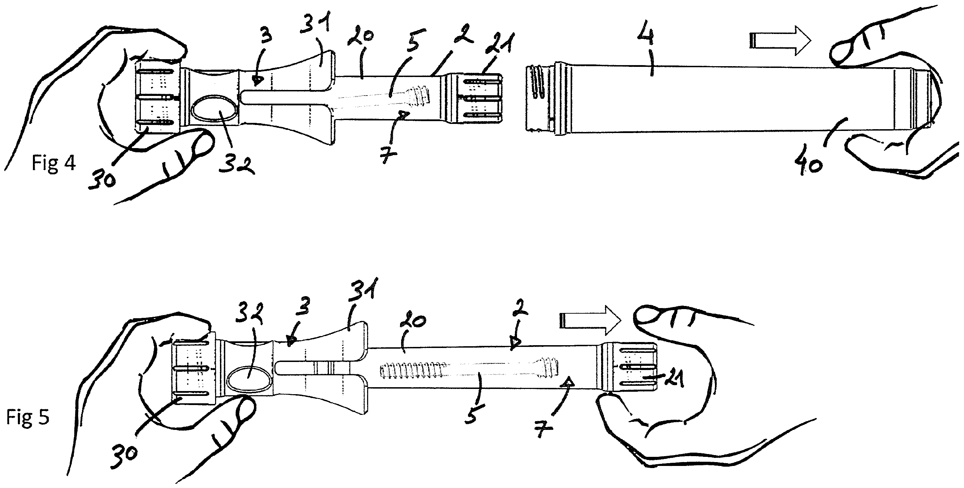

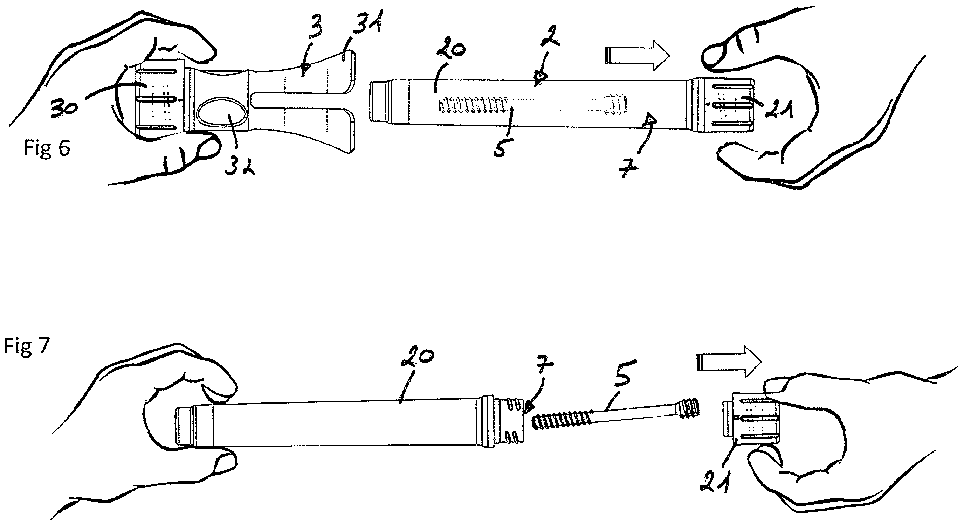

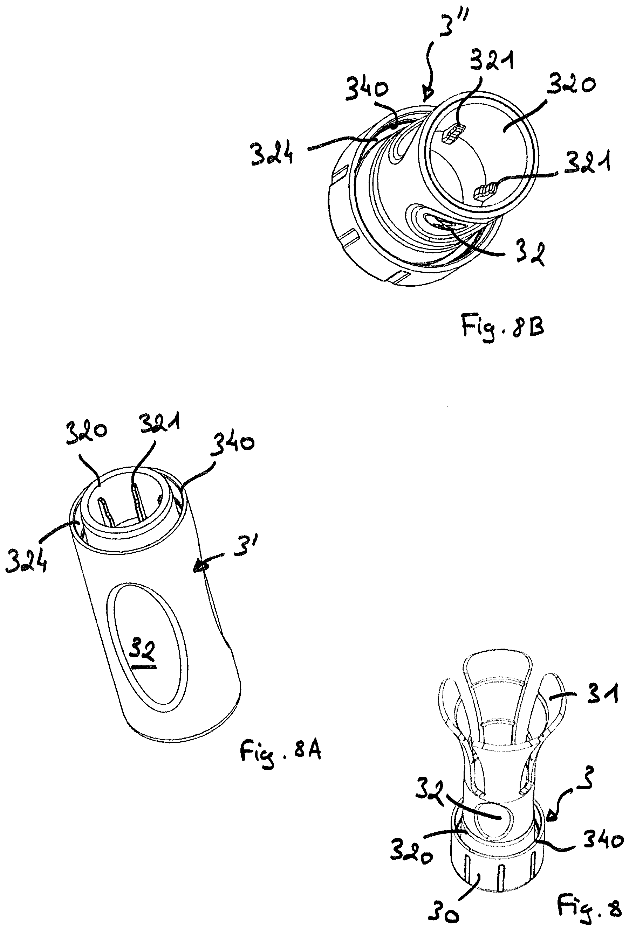

Ledit bouchon 3, et éventuellement la coiffe 4, présente(nt) au moins deux emplacements 32 prédéfinis permettant de positionner chacun au moins un doigt d'une personne pour lui permettre de saisir ledit bouchon ou ladite coiffe par pincement entre au moins deux doigts. Préférentiellement, au moins deux desdits emplacements 32 prédéfinis sont diamétralement opposés par rapport à l'axe de l'élément, bouchon 3 ou coiffe 4, sur lequel ils sont ménagés. Dans l'exemple illustré aux figures 1 à 8 et 8B, chaque emplacement 32 est ménagé sur une portion du bouchon destinée à être recouverte par la coiffe 4. Dans ce cas, ledit emplacement devient accessible une fois la coiffe retirée.

Selon un autre mode de réalisation du bouchon 3' illustré à la figure 8A, ledit emplacement 32 est ménagé sur une portion du bouchon 3' qui n'est pas destinée à être recouverte par la coiffe.

Avantageusement, le bouchon 3 ; 3' présente une longueur suffisante pour permettre à la personne de positionner ses doigts sur ledit bouchon, et en particulier de saisir le bouchon avec un pouce, sans risque de toucher l'élément support 2.

Lesdits éléments de support 2 et de protection 3, 4 de l'emballage s'emboitent à recouvrement partiel entre eux par friction et/ou par vissage. Les moyens de couplage des éléments les uns par rapport aux autres sont détaillés ci-après.

Dans l'exemple illustré aux figures, le bouchon 3 et la coiffe 4 s'emboitent entre eux par vissage, de même que les parties 20, 21 qui composent l'élément support 2. Préférentiellement, le filet et le taraudage ménagés sur les éléments qui s'emboitent sont configurés de manière à ne permettre le vissage desdits éléments entre eux qu'une seule fois. En particulier, on peut prévoir que le filetage et/ou le taraudage se déforment lors du vissage et du dévissage de sorte qu'un nouveau vissage des deux éléments entre eux n'est plus possible.

En variante, l'emboitement à recouvrement partiel desdits éléments de l'emballage pourrait être réalisé par simple friction, par exemple par pression ou serrage élastique. Un tel emboitement par simple friction permet de procéder au déboitement desdits éléments par simple traction de l'une ou de chacune des personnes sur un élément de l'emballage selon un geste naturel. On pourrait également envisager un emboîtement par clipsage.

Dans l'exemple illustré aux figures 1 à 8 et 8B, ledit bouchon 3 présente aussi au moins une partie 31, de préférence au moins une ailette, déformable, permettant d'emboiter partiellement ledit bouchon à l'intérieur de la coiffe. Ladite partie 31 est configurée pour, d'une part, à l'état recouvert de ladite partie 31 déformable du bouchon par la coiffe, venir en appui contre la face interne de coiffe 4 afin de permettre un couplage du bouchon à la coiffe par friction et, d'autre part, pour pouvoir se déformer afin de permettre la séparation de la coiffe 4 du bouchon 3.

Ledit bouchon 3 est formé par un corps creux ouvert à une extrémité et fermée à l'autre extrémité. La coiffe 4 est aussi formée par un corps creux ouvert à une extrémité et fermée à l'autre extrémité. L'extrémité fermée du bouchon 3 définie une partie de fond du bouchon. Comme détaillé ci-après, ledit bouchon 3 comprend des moyens de couplage avec l'élément support 2.

Ledit bouchon 3 comprend un corps sensiblement de révolution autour d'un axe correspondant à l'axe longitudinal dudit bouchon 3. Le corps du bouchon comprend une paroi périphérique 320 dite interne, et une paroi périphérique 340 dite externe qui entoure ladite paroi périphérique 320 interne. La paroi périphérique 340 externe est sensiblement coaxiale à la paroi périphérique 320 interne.

Ladite paroi périphérique 340 externe entoure à écartement ladite paroi périphérique 320 interne pour définir entre lesdites parois périphériques un espace annulaire 324 d'insertion de la partie de la coiffe 4 destinée à être couplée audit bouchon 3. Ladite partie de la coiffe 4 destinée à être couplée audit bouchon 3 correspond à l'extrémité ouverte de la coiffe 4.

On peut prévoir de ménager sur les parois périphériques externe 340 et/ou interne 320 des moyens de vissage, tels que taraudage et/ou filetage, aptes à coopérer avec des moyens de vissage complémentaires, tels que filetage et/ou taraudage, ménagés sur la paroi périphérique de la coiffe 4.

La face interne de la paroi périphérique 340 dite externe et/ou la face externe de la paroi périphérique 320 dite interne peu(ven)t présenter un taraudage et/ou un filetage pour coopérer avec un filet et/ou un taraudage complémentaire ménagé(s) sur la face périphérique externe et/ou la face périphérique interne du corps de la coiffe 4.

Dans l'exemple illustré aux figures, la face interne de la paroi périphérique 340 externe présente un taraudage apte à coopérer avec un filet ménagé sur la face externe de la paroi périphérique de la coiffe 4.

On peut également prévoir que l'écartement entre les parois périphériques externe 340 et interne 320 du bouchon soit configuré de telle sorte que l'emboitement de la coiffe 4 avec le bouchon 3 génère un pincement de la paroi périphérique de la coiffe 4 entre les parois périphériques externe 340 et interne 320 du bouchon.

Dans l'exemple illustré aux figures, le bouchon 3 et l'élément support 2 s'emboitent par enfoncement à force, c'est-à-dire par pression d'une partie d'extrémité de l'élément support 2 dans une partie creuse correspondante du bouchon 3.

Autrement dit, la face interne de la paroi périphérique interne 320 du bouchon 3 présente des moyens de maintien 321 permettant de maintenir la partie de l'élément support 2 emboitée à force à l'intérieur de l'espace délimité par la paroi périphérique interne 320 du bouchon 3.

Lesdits moyens de maintien 321 comprennent des ergots qui sont destinés à être en contact d'appui avec l'élément support 2 à l'état enfoncé dudit élément support dans le bouchon 3. On obtient ainsi un bon maintien de l'élément support 2 avec le bouchon 3 avant leur séparation en vue d'ouvrir ladite première chambre 7. En particulier, chaque ergot présente une partie formant rampe configurée de sorte que l'élément support 2 qui est emboité dans la paroi périphérique interne 320 pousse radialement les ergots vers l'extérieur et donc déforme la paroi périphérique interne 320 correspondante vers la paroi périphérique externe 340.

Plus précisément, comme illustré plus particulièrement aux figures 8A et 8B, les ergots 321 sont répartis sur la face périphérique interne du bouchon, autour de l'axe dudit bouchon 3, de préférence en étant espacés angulairement les uns des autres.

La présence desdits ergots à l'intérieur de la paroi périphérique interne 320 du bouchon permet de maintenir à force l'élément support 2 emboité dans le bouchon 3, tout en bénéficiant lors d'un tel emboitement de l'élément support 2 dans la paroi périphérique interne du bouchon, d'un rapprochement de la paroi périphérique interne 320 du bouchon par rapport à la paroi périphérique externe 340 dudit bouchon provoqué par appui de l'élément support 2 sur les ergots 321.

Le rapprochement entre elles par déformation élastique des parois périphériques interne 320 et externe 340 permet de renforcer l'étanchéité de la liaison au niveau de l'emboitement entre la coiffe 4 et le bouchon 3. En effet, les parois périphériques 320, 340 peuvent ainsi pincer la paroi périphérique de la partie du bouchon 3 emboitée dans l'espace ménagé entre lesdites parois périphériques interne 320 et externe 340 du bouchon.

Ainsi, lesdits moyens de maintien 321 ménagés à l'intérieur de la paroi périphérique interne 320 du bouchon 3 permettent d'améliorer non seulement le maintien de l'élément support 2 par rapport au bouchon 3, mais également l'étanchéité entre le bouchon 3 et la coiffe 4 dont la paroi périphérique est pincée entre lesdites parois périphériques interne 320 et externe 340 du bouchon 3.

Dans l'exemple illustré aux figures, le maintien entre la coiffe 4 et le bouchon 3 est assuré par les moyens de vissage complémentaires de type filetage et taraudage ménagés sur la coiffe 4 et le bouchon 3, et l'étanchéité entre la coiffe 4 et le bouchon 3 est obtenue par pincement de la paroi périphérique de la coiffe 4 entre lesdites parois périphériques 320 et 340 du bouchon 3 résultant de l'emboitement de l'élément support 2 dans le bouchon 3 qui déforme élastiquement la paroi périphérique 320 vers la paroi périphérique 340 du bouchon 3. Le maintien de l'élément support 2 dans le bouchon 3 est assuré par les ergots 321 qui enserrent l'élément support 2.

En outre, les ergots 321 forment des entretoises entre la paroi périphérique 320 et l'élément support 2 qui permettent de maintenir un écartement radial entre l'élément support 2 et la paroi périphérique 320. En outre, l'espacement entre les ergots 321 permet de définir un espace de communication de fluide entre l'intérieur de la paroi périphérique 320 et l'intérieur de la coiffe 4.

Un tel passage de communication entre l'intérieur de la coiffe 4 et l'intérieur de la paroi périphérique 320 permet à un gaz de stérilisation injecté dans la coiffe 4, par exemple via une membrane perméable au gaz, et de préférence imperméable aux bactéries, de se répandre à l'intérieur du volume délimité par la paroi périphérique 320 pour stériliser les deux chambres 7 et 8 de l'emballage, notamment lorsque l'élément support 2 est un élément ouvert ou lorsque l'extrémité de l'élément support 2 emboitée dans la paroi périphérique 320, présente une membrane perméable au gaz, et de préférence imperméable aux bactéries.

On peut prévoir que les moyens de maintien 321 de l'élément support 2 par rapport au bouchon 3 comprennent également des moyens de vissage, par exemple de type taraudage, aptes à coopérer avec des moyens de vissage complémentaires, par exemple de type filetage, ménagés sur la face externe de la paroi périphérique de l'élément support 2.

La combinaison de moyens de vissage et d'ergots permet ainsi de combiner un maintien de l'élément support 2 par rapport au bouchon 3 par vissage et également par friction en conservant les avantages de la liaison entre le bouchon 3 et la coiffe 4 rappelés ci-dessus.

En outre, dans l'exemple illustré aux figures, la paroi périphérique interne 320 s'étend en saillie de la paroi périphérique externe 340 du côté opposé au fond du bouchon 3.

Selon les variantes de réalisation de bouchon illustrées aux figures 8A et 8B, les bouchons 3' ; 3" présentent également chacun une partie creuse, munie d'ergots, dans laquelle est destinée à être enfoncée une partie d'extrémité de l'élément support correspondant.

L'élément support 2 et/ou le bouchon 3 et/ou la coiffe 4 présentent une partie, de préférence formée par une membrane, imperméable aux bactéries mais perméable aux gaz pour permettre une stérilisation gazeuse de ladite première chambre 7 et/ou de ladite deuxième chambre 8.

L'emballage décrit ci-dessus permet de mettre en oeuvre un procédé de déconditionnement qui comprend les étapes suivantes. Ce procédé est décrit en référence aux figures 1 à 8 pour lesquelles l'élément support est formé de deux pièces couplables/désaccouplables de manière à délimiter une cavité fermée qui renferme ledit objet. Comme illustré aux figures 1 à 4, une première personne, supposée en zone contaminée, sépare la coiffe 4 par rapport au bouchon 3 de manière à découvrir l'élément support 2 qui reste écarté des mains de la personne supposée contaminée grâce au fait que la main de ladite personne tient l'emballage restant par le bouchon 3, c'est-à-dire à écartement de l'élément support. La personne contaminée peut alors tendre à une deuxième personne, supposée en zone propre, l'emballage restant du côté de l'élément support 2. Ladite personne propre peut alors tirer sur l'élément support 2 pour entrainer la séparation dudit élément support 2 par rapport au bouchon 3 tenu par la première personne supposée contaminée. Ladite personne "propre" est par exemple le chirurgien au niveau du bloc opératoire.

Eventuellement, l'élément support 2 qui renferme l'objet 5 peut être mis dans une zone d'attente, en attente de l'utilisation dudit objet 5. Puis ladite deuxième personne ou une autre personne, supposée propre, peut ouvrir la première chambre 7 définie par ledit élément support 2 en séparant, ici par dévissage, les deux pièces 20, 21 qui forment ledit élément support pour saisir ladite pièce médicale.

Dans le cas non illustré aux figures, où l'élément support forme directement la première chambre qui loge ledit objet en coopération avec le bouchon, le procédé décrit ci-dessus est adapté en prévoyant que la personne contaminée tende à la deuxième personne, supposée en zone propre, l'emballage restant du côté de l'élément support 2, en orientant ledit emballage restant de telle sorte que l'élément support 2 se retrouve à une hauteur inférieure à celle du bouchon 3, de sorte que ledit objet 5 reste contenu dans l'élément support 2 lors de la séparation dudit élément support 2 par rapport au bouchon 3 pour éviter que ledit objet 5 ne tombe par terre.

Ainsi, la pièce médicale n'a été ni touchée ni lâchée au cours de son déconditionnement. En outre, la chambre contenant la pièce médicale n'est ouverte qu'en zone propre.

Grâce à la coiffe qui délimite une chambre de protection de la partie de saisie 20 de l'élément support 2, la personne propre touche une partie propre de l'emballage. En effet, les parties sales de l'emballage, à savoir le bouchon 3 et la coiffe 4, sont restées dans les mains de la personne sale. Ainsi, on est assuré que la main de la personne qui saisit l'élément support reste propre.

Bien entendu dans chacun des modes de réalisation on peut prévoir que la séparation de la coiffe et du bouchon soit réalisée par deux personnes différentes en zone sale au lieu d'une seule, l'une tenant une extrémité du bouchon et l'autre une extrémité de la coiffe.

Préférentiellement, les chambres 7 et 8 sont imperméables au moins aux bactéries. On peut prévoir qu'elles soient également imperméables à tout fluide. En variante, on peut prévoir que l'une et/ou l'autre, de préférence au moins la chambre de présentation 7 de l'objet, soit perméable uniquement aux gaz pour permettre une stérilisation gazeuse de la ou des chambres.

Ainsi, on peut prévoir, selon un mode de réalisation particulier de l'invention, qu'au moins une partie de la paroi du bouchon 3 qui délimite une partie de la chambre 7 de présentation de l'objet 5 est formée en matériau perméable au gaz, mais imperméable aux bactéries de manière à permettre une stérilisation gazeuse de la chambre de présentation et de l'objet, par exemple une pièce médicale, par exemple avec de l'oxyde d'éthylène ou par vapeur. Une telle conception de l'emballage permet de stériliser la ou les chambres autrement que par rayonnement.

On peut ainsi prévoir que le bouchon soit fermé à son extrémité, opposée à celle qui recouvre partiellement l'élément support, par une membrane adaptée à la stérilisation gazeuse.

La présente invention n'est nullement limitée aux modes de réalisation décrits et représentés, mais l'homme du métier saura y apporter toute variante conforme aux revendications.

## Revendications

1. Ensemble médical, de préférence stérilisé, comprenant un objet (5) médical et un emballage (1) contenant ledit objet (5),
ledit emballage comprenant un élément support (2) creux à l'intérieur duquel ledit objet (5) est libre,
ledit emballage comprenant :
- un premier élément de protection (3), appelé bouchon, couplable à l'élément support (2) dudit objet (5), de préférence par emboitement à recouvrement partiel, de telle sorte que l'élément support (2) présente une partie (20) de saisie qui s'étend en saillie dudit bouchon (3),
ledit élément support (2) délimitant à lui seul ou en coopération avec le bouchon (3) une première chambre (7) renfermant ledit objet (5),
- un deuxième élément de protection (4) creux, appelé coiffe,
ladite coiffe (4) étant couplable au bouchon (3), de préférence par emboîtement à recouvrement partiel, de manière à délimiter en coopération avec ledit bouchon (3) une deuxième chambre (8) à l'intérieur de laquelle s'étend la partie de saisie (20) de l'élément support (2),
et, à l'état couplé de la coiffe (4) au bouchon (3), une partie du bouchon (3) s'étendant en saillie de la coiffe (4),
ledit bouchon (3) comprenant une paroi périphérique (320) dite interne, et une paroi périphérique (340) dite externe qui entoure à écartement ladite paroi périphérique (320) interne pour définir entre lesdites parois périphériques (320, 340) un espace annulaire (324) d'emboitement de la partie de la coiffe (4) destinée à être couplée audit bouchon (3), la paroi périphérique (320) interne définissant un espace d'emboitement de la partie de l'élément support (2) destinée à être couplée audit bouchon (3),
en ce que la face interne de la paroi périphérique interne (320) du bouchon (3) est munie de moyens de maintien (321) permettant de maintenir la partie de l'élément support (2) emboitée à l'intérieur de l'espace délimité par ladite paroi périphérique interne (320) du bouchon (3),
**caractérisé en ce que** lesdits moyens de maintien (321) comprennent des ergots qui sont destinés à être en contact d'appui avec l'élément support (2) à l'état emboité dudit élément support (2) dans le bouchon (3),
et **en ce que** les ergots (321) sont répartis sur la face périphérique interne (320) du bouchon (3), autour de l'axe dudit bouchon (3), en étant espacés angulairement les uns des autres.

2. Ensemble médical selon la revendication 1, **caractérisé en ce que** chaque ergot (321) comprend une partie formant rampe, dirigée vers l'axe du bouchon (3) en partant d'un point bas de la rampe situé du côté de l'extrémité ouverte du bouchon (3) vers un point haut de ladite rampe situé du côté de l'extrémité fermée dudit bouchon (3), de sorte que, lors de l'emboitement de l'élément support (2) à l'intérieur de l'espace défini par la paroi périphérique interne (320) du bouchon (3), ledit élément support (2), par appui sur ladite rampe, pousse radialement ledit ergot (321) et déforme la paroi périphérique interne (320) correspondante vers la paroi périphérique externe (340).

3. Ensemble médical selon la revendication 1 ou 2, **caractérisé en ce que** les ergots (321) forment des entretoises permettant de maintenir un écartement radial entre l'élément support (2) et la paroi périphérique interne (320) du bouchon (3), l'espacement entre les ergots (321) définissant un espace de communication de fluide entre l'intérieur de la paroi périphérique (320) du bouchon (3) et l'intérieur de la coiffe (4).

4. Ensemble médical selon l'une des revendications précédentes, **caractérisé en ce que** lesdites parois périphériques externe (340) et/ou interne (320) du bouchon (3) comprennent des moyens de vissage aptes à coopérer avec des moyens de vissage complémentaires ménagés sur la coiffe (4).

5. Ensemble médical selon la revendication 4, **caractérisé en ce que** la face interne de la paroi périphérique (340) du bouchon (3) externe présente un taraudage apte à coopérer avec un filet ménagé sur la face externe de la paroi périphérique de la coiffe (4).

6. Ensemble médical selon l'une des revendications précédentes, **caractérisé en ce que**, ledit élément support (2) délimitant à lui seul ladite première chambre (7) renfermant ledit objet (5), ledit élément support (2) est formé d'au moins deux pièces (20, 21) couplables/désaccouplables l'une par rapport à l'autre, de préférence par emboitement à recouvrement partiel, pour former ladite première chambre renfermant ledit objet (5).

7. Ensemble médical selon l'une des revendications 1 à 5, **caractérisé en ce que**, ledit élément support (2) délimitant en coopération avec le bouchon (3) ladite première chambre (7) renfermant ledit objet (5), ledit élément support (2) délimite une cavité ouverte destinée à être refermée par ledit bouchon (3).

8. Ensemble médical selon l'une des revendications précédentes, **caractérisé en ce que** ledit bouchon (3), et éventuellement la coiffe (4), présente(nt) des emplacements prédéfinis (32) permettant de positionner au moins deux doigts, de préférence trois doigts, d'une personne pour lui permettre de saisir ledit bouchon, et éventuellement ladite coiffe, au niveau desdits emplacements prédéfinis.

9. Ensemble médical selon l'une des revendications précédentes, **caractérisé en ce que** l'élément support (2) et/ou le bouchon (3) et/ou la coiffe (4) présente(nt) une partie, de préférence formée par une membrane, imperméable aux bactéries mais perméable aux gaz pour permettre une stérilisation gazeuse de ladite première chambre (7) et/ou de ladite deuxième chambre (8).

10. Procédé de déballage d'un objet médical contenu dans un emballage (1) d'ensemble médical, de préférence stérilisé, conforme à l'une des revendications précédentes, ledit procédé comprenant les étapes suivantes :
a)- séparation de la coiffe (4) par rapport au bouchon (3) par une première personne de manière à découvrir l'élément support (2),
b)- saisie par une deuxième personne de l'élément support (2),
c)- séparation dudit élément support (2) par rapport au bouchon (3) tenu par la première personne.

11. Procédé de déballage selon la revendication 10, ledit emballage étant conforme à la revendication 6, **caractérisé en ce que** ledit procédé comprend les étapes supplémentaires suivantes :
d)- éventuellement, mise en attente dudit élément support (2) qui renferme l'objet (5) dans une zone d'attente, en attente de l'utilisation dudit objet (5),
e)- ouverture de la première chambre (7) définie par ledit élément support (2) pour en extraire ledit objet (5).

12. Procédé de déballage selon la revendication 10, ledit emballage étant conforme à la revendication 7, **caractérisé en ce que** ledit procédé comprend entre les étapes a) et b) l'étape supplémentaire suivante de présentation, par la première personne à la deuxième personne, de l'emballage restant, formé du bouchon (3) et de l'élément support (2), en lui présentant l'extrémité dudit emballage restant formée par ledit élément support (2), en orientant ledit emballage restant de telle sorte que l'élément support (2) se retrouve à une hauteur inférieure à celle du bouchon (3) de sorte que ledit objet (5) reste contenu dans l'élément support (2) lors de la séparation dudit élément support (2) par rapport au bouchon (3) pour éviter que ledit objet (5) ne tombe par terre.

## Patentansprüche

1. Medizinische Anordnung, vorzugsweise sterilisiert, umfassend einen medizinischen Gegenstand (5) und eine Verpackung (1), die den Gegenstand (5) enthält,
wobei die Verpackung ein hohles Trägerelement (2) umfasst, in dem der Gegenstand (5) frei ist,
wobei die Verpackung umfasst:
- ein erstes Trägerelement (3), genannt Deckel, das mit dem Trägerelement (2) des Gegenstands (5), vorzugsweise durch Eingriff mit teilweiser Überlappung, derart gekoppelt werden kann, dass das Trägerelement (2) ein Griffteil (20) aufweist, das aus dem Deckel (3) herausragt,
das Trägerelement (2) allein oder in Zusammenarbeit mit dem Deckel (3) eine erste Kammer (7) begrenzt, die den Gegenstand (5) umschließt,
- ein zweites hohles Schutzelement (4), genannt Abdeckung,
wobei die Abdeckung (4) mit dem Deckel (3), vorzugsweise durch Eingriff mit teilweiser Überlappung, so gekoppelt werden kann, dass sie in Zusammenarbeit mit dem Deckel (3) eine zweite Kammer (8) begrenzt, in der sich das Griffteil (20) des Trägerelements (2) erstreckt,
und, in dem Zustand, in dem die Abdeckung (4) mit dem Deckel (3) gekoppelt ist, ein Teil des Deckels (3), der aus der Abdeckung (4) herausragt,
wobei der Deckel (3) eine Umfangswand (320), genannt Innenumfangswand, und eine Umfangswand (340), genannt Außenumfangswand, die die Innenumfangswand (320) mit Abstand umgibt, umfasst, um zwischen den Umfangswänden (320, 340) einen Ringraum (324) für den Eingriff des Teils der Abdeckung (4) zu definieren, der mit dem Deckel (3) verbunden werden soll, die Innenumfangswand (320), die einen Eingriffsraum für den Teil des Trägerelements (2) definiert, der mit dem Deckel (3) gekoppelt werden soll, wobei die Innenfläche der Innenumfangswand (320) des Deckels (3) mit Haltemitteln (321) zum Halten des Teils des Trägerelements versehen ist, das in den Raum eingreift, der durch die innere Umfangswand (320) des Deckels (3) begrenzt ist,
**dadurch gekennzeichnet, dass** die Haltemittel (321) Laschen aufweisen, die dazu bestimmt sind, in dem Zustand, in dem das Trägerelement (2) in den Deckel (3) eingreift, mit dem Trägerelement (2) in Kontakt zu stehen,
und dass die Laschen (321) auf der Innenumfangsfläche (320) des Deckels (3) um die Achse des Deckels (3) verteilt sind, indem sie voneinander winklig beabstandet sind.

2. Medizinische Anordnung nach Anspruch 1, **dadurch gekennzeichnet, dass** jede Lasche (321) einen rampenbildenden Teil umfasst, der ausgehend von einem unteren Punkt der Rampe am offenen Ende des Deckels (3) bis zu einem oberen Punkt der Rampe am geschlossenen Ende des Deckels (3), derart auf die Achse des Deckels (3) gerichtet ist, dass, wenn das Trägerelement (2) innerhalb des durch die Innenumfangswand (320) des Deckels (3) definierten Raumes in Eingriff steht, das Trägerelement (2) die Lasche (321) durch Auflegen auf die Rampe radial drängt und die entsprechende Innenumfangswand (320) in Richtung der Außenumfangswand (340) verformt.

3. Medizinische Anordnung nach Anspruch 1 oder 2, **dadurch gekennzeichnet, dass** die Laschen (321) Abstandshalter zum Aufrechterhalten eines radialen Abstandes zwischen dem Trägerelement (2) und der Innenumfangswand (320) des Deckels (3) bilden, wobei der Abstand zwischen den Laschen (321) einen Raum für eine Fluidverbindung zwischen der Innenseite der Umfangswand (320) des Deckels (3) und der Innenseite der Abdeckung (4) definiert.

4. Medizinische Anordnung nach einem der vorstehenden Ansprüche, **dadurch gekennzeichnet, dass** die Außen-(340) und/oder Innenumfangswände (320) des Deckels (3) Schraubmittel zum Zusammenwirken mit komplementären Schraubmitteln umfassen, die auf dem Deckel (4) ausgebildet sind.

5. Medizinische Anordnung nach Anspruch 4, **dadurch gekennzeichnet, dass** die Innenfläche der Außenumfangswand (340) des Anschlags (3) eine Gewindebohrung aufweist, die mit einem an der Außenfläche der Umfangswand der Abdeckung (4) ausgebildeten Gewinde zusammenwirken kann.

6. Medizinische Anordnung nach einem der vorhergehenden Ansprüche, **dadurch gekennzeichnet, dass**, wenn das Trägerelement (2) die erste Kammer (7), die den Gegenstand (5) umschließt, allein begrenzt, das Trägerelement (2) aus mindestens zwei Komponenten (20, 21) gebildet ist, die miteinander verbunden/entkoppelt werden können, vorzugsweise durch Eingriff mit teilweiser Überlappung, um die erste Kammer zu bilden, die den Gegenstand (5) umschließt.

7. Medizinische Anordnung nach einem der Ansprüche 1 bis 5, **dadurch gekennzeichnet, dass**, wenn das Trägerelement (2) in Zusammenarbeit mit dem Deckel (3) die erste Kammer (7), die den Gegenstand (5) umschließt, begrenzt, das Trägerelement (2) einen offenen Hohlraum begrenzt, der dazu bestimmt ist, durch den Deckel (3) verschlossen zu werden.

8. Medizinische Anordnung nach einem der vorstehenden Ansprüche, **dadurch gekennzeichnet, dass** der Deckel (3), und gegebenenfalls die Abdeckung (4), vordefinierte Stellen (32) aufweist (aufweisen), die die Positionierung von mindestens zwei Fingern, vorzugsweise drei Fingern, einer Person ermöglichen, um es ihr zu ermöglichen, den Deckel und gegebenenfalls die Abdeckung im Bereich der vordefinierten Stellen zu greifen.

9. Medizinische Anordnung nach einem der vorstehenden Ansprüche, **dadurch gekennzeichnet, dass** das Trägerelement (2) und/oder der Deckel (3) und/oder die Abdeckung (4) einen Teil aufweisen, der vorzugsweise aus einer Membran besteht, der für Bakterien undurchlässig, aber für Gase durchlässig ist, um eine Gassterilisation der ersten Kammer (7) und/oder der zweiten Kammer (8) zu ermöglichen.

10. Verfahren zum Auspacken eines medizinischen Gegenstands, der in einer Verpackung (1) einer medizinischen Anordnung, vorzugsweise sterilisiert, nach einem der vorhergehenden Ansprüche, enthalten ist, wobei das Verfahren die folgenden Schritte umfasst:
a) Trennen der Abdeckung (4) vom Deckel (3) durch eine erste Person, um das Trägerelement (2) freizulegen,
b) Greifen des Trägerelements (2) durch eine zweite Person,
c) Trennen des Trägerelements (2) von dem Deckel (3), der von der ersten Person gehalten wird.

11. Verfahren zum Auspacken nach Anspruch 10, wobei die Verpackung gemäß Anspruch 6 ausgebildet ist, **dadurch gekennzeichnet, dass** das Verfahren die folgenden zusätzlichen Schritte umfasst:
d) gegebenenfalls, Bereithalten des Trägerelements (2), das den Gegenstand (5) umschließt, in einem Bereithaltebereich bis zur Verwendung des Gegenstands (5),
e) Öffnen der ersten Kammer (7), die durch das Trägerelement (2) definiert ist, um den Gegenstand (5) daraus zu extrahieren.

12. Verfahren zum Auspacken nach Anspruch 10, wobei die Verpackung gemäß Anspruch 7 ausgebildet ist, **dadurch gekennzeichnet, dass** das Verfahren zwischen den Schritten a) und b) den folgenden zusätzlichen Schritt umfasst, eines Präsentierens durch die erste Person der verbleibenden Verpackung, die aus dem Deckel (3) und dem Trägerelement (2) besteht, der zweiten Person, indem sie ihr das Ende der verbleibenden Verpackung, die aus dem Trägerelement (2) gebildet wird, präsentiert, indem sie die verbleibende Verpackung derart ausrichtet, dass sich das Trägerelement (2) auf einer Höhe befindet, die niedriger ist als die des Deckels (3), so dass der Gegenstand (5) während der Trennung des Trägerelements (2) vom Deckel (3) im Trägerelement (2) enthalten bleibt, um zu vermeiden, dass der Gegenstand (5) auf den Boden fällt.

## Claims

1. Medical assembly, preferably a sterilized one, comprising a medical article (5) and a package (1) containing said article (5),
said package comprising a hollow support element (2) inside which said article (5) is free,
said package comprising:
- a first protective element (3), called a stopper, which can be coupled to the support element (2) of said article (5), preferably by engagement with partial overlap, in such a way that the support element (2) has a grip part (20) that protrudes from said stopper (3),
said support element (2) delimiting on its own, or in cooperation with the stopper (3), a first chamber (7) that encloses said article (5),
- a second hollow protective element (4), called a cover,
said cover (4) being able to be coupled to the stopper (3), preferably by engagement with partial overlap, in such a way as to delimit, in cooperation with said stopper (3), a second chamber (8) inside which there extends the grip part (20) of the support element (2),
and, in the state when the cover (4) is coupled to the stopper (3), a part of the stopper (3) protruding from the cover (4) said stopper (3) comprising a peripheral wall (320) called an internal wall, and a peripheral wall (340) called an external wall which surrounds said internal peripheral wall (320) with clearance in order to define, between said peripheral walls (320, 340), an annular space (324) for engagement of that part of the cover (4) intended to be coupled to said stopper (3), the internal peripheral wall (320) defining a space for engagement of that part of the support element (2) intended to be coupled to said stopper (3) in that the internal face of the internal peripheral wall (320) of the stopper (3) is provided with retaining means (321) for retaining that part of the support element (2) engaged inside the space delimited by said internal peripheral wall (320) of the stopper (3) **characterized in that** said retaining means (321) comprise lugs which are intended to be in bearing contact with the support element (2) in the state when said support element (2) is engaged in the stopper (3),
and **in that** the lugs (321) are distributed on the internal peripheral face (320) of the stopper (3), about the axis of said stopper (3), by being spaced apart at angles from one another.

2. Medical assembly according to Claim 1, **characterized in that** each lug (321) comprises a ramp-forming part, directed toward the axis of the stopper (3) starting from a bottom point of the ramp situated at the open end of the stopper (3) to a top point of said ramp situated at the closed end of said stopper (3), in such a way that, when the support element (2) is engaged inside the space defined by the internal peripheral wall (320) of the stopper (3), said support element (2), by bearing on said ramp, pushes said lug (321) radially and deforms the corresponding internal peripheral wall (320) toward the external peripheral wall (340).

3. Medical assembly according to Claim 1 or 2, **characterized in that** the lugs (321) form spacers for maintaining a radial distance between the support element (2) and the internal peripheral wall (320) of the stopper (3), the spacing between the lugs (321) defining a space for fluid communication between the inside of the peripheral wall (320) of the stopper (3) and the inside of the cover (4).

4. Medical assembly according to one of the preceding claims, **characterized in that** said external (340) and/or internal (320) peripheral walls of the stopper (3) comprise screwing means for cooperating with complementary screwing means formed on the cover (4).

5. Medical assembly according to Claim 4, **characterized in that** the internal face of the external peripheral wall (340) of the stopper (3) has a tapping that is able to cooperate with a thread formed on the external face of the peripheral wall of the cover (4).

6. Medical assembly according to one of the preceding claims, **characterized in that**, when said support element (2) delimits on its own said first chamber (7) enclosing said article (5), said support element (2) is formed by at least two components (20, 21) that can be coupled to/uncoupled from each other, preferably by engagement with partial overlap, in order to form said first chamber enclosing said article (5).

7. Medical assembly according to one of Claims 1 to 5, **characterized in that**, when said support element (2) delimits, in cooperation with the stopper (3), said first chamber (7) enclosing said article (5), said support element (2) delimits an open cavity intended to be closed by said stopper (3).

8. Medical assembly according to one of the preceding claims, **characterized in that** said stopper (3), and if appropriate the cover (4), has (have) predefined locations (32) permitting the positioning of at least two fingers, preferably three fingers, of a person in order to allow him to grip said stopper, and if appropriate said cover, in the area of said predefined locations.

9. Medical assembly according to one of the preceding claims, **characterized in that** the support element (2) and/or the stopper (3) and/or the cover (4) have a part, preferably formed by a membrane, that is impermeable to bacteria but permeable to gases in order to permit gas sterilization of said first chamber (7) and/or of said second chamber (8).

10. Method of unpacking a medical article contained in a package (1) of a medical assembly, preferably a sterilized one, according to one of the preceding claims, said method comprising the following steps:
a) separation of the cover (4) from the stopper (3) by a first person in order to uncover the support element (2),
b) gripping of the support element (2) by a second person,
c) separation of said support element (2) from the stopper (3) held by the first person.

11. Method of unpacking according to Claim 10, said package being according to Claim 6, **characterized in that** said method comprises the following additional steps:
d) optionally keeping said support element (2), which encloses the article (5), in a stand-by area pending the use of said article (5),
e) opening the first chamber (7) defined by said support element (2) in order to remove said article (5) therefrom.

12. Method of unpacking according to Claim 10, said package being according to Claim 7, **characterized in that** said method comprises, between steps a) and b), the following additional step in which the first person offers the second person the remaining package composed of the stopper (3) and of the support element (2), by presenting to him the end of said remaining package formed by said support element (2), orienting said remaining package in such a way that the support element (2) is at a height lower than that of the stopper (3), such that said article (5) remains contained in the support element (2) during the separation of said support element (2) from the stopper (3), so as to avoid said article (5) falling to the ground.
